# EUROPEAN PATENT SPECIFICATION

(11) **EP 2 257 260 B1**
(45) Date of publication and mention of the grant of the patent: **25.01.2017**
(21) Application number: 09727929.3
(22) Date of filing: 01.04.2009
(51) Int. Cl.: A61H 15/00, A47G 9/02, A61M 21/02

(54) **WEIGHT QUILT**
WINTERDECKE
COUVERTURE MATELASSÉE DE POIDS

(30) Priority: 02.04.2008 SE 0800742
(43) Date of publication of application: 08.12.2010
(73) Proprietor: Somna AB, 443 61 Stenkullen (SE)
(72) Inventor: RAMER, Staffan, S-436 51 Hovas (SE)
(74) Representative: Johansson, Lars-Erik
(86) International application number: PCT/SE2009/050344
(87) International publication number: WO 2009/123557

(56) References cited:
- WO-A1-95/13045
- JP-A- 11 123 131
- JP-A- H11 123 131
- US-A- 1 269 316
- US-A- 1 364 601
- US-A1- 2006 016 005
- US-A1- 2006 174 410
- US-A1- 2007 028 387

## Description

### TECHNICAL FIELD

The present invention concerns a weight blanket for therapeutic treatment, said blanket comprising a plurality of linked objects for increasing the weight of the blanket; said linked objects being placed in several corresponding channels between a first and a second cloth layer in the blanket, said channels being distributed over the surface of the blanket.

### STATE OF TECHNOLOGY

It has been known for a long.time that persons who suffer from angst, unrest, or sensory disorders have difficulty in finding rest, sleeping, or relaxing. A reason for this may be that they have difficulty in feeling the boundaries of their own bodies, and they can need a physical object to focus on, preferably something that presses against the body and that they can bend and wrap around themselves, perhaps a pillow or a blanket with a certain weight. Especially for hyperactive, restless children or children with an ADHD problem or Asperger's syndrome, such an article makes a great difference when calm, sleep, or insufficient relaxation is involved, but also children and adults with a general complaint of sleep problems, unrest, and angst can benefit from a simple and practical solution to relieve the complaint.

By pressure applied to the body, the user receives a feeling of safety, and it is believed that a number of bodily substances are released that have a calming effect, including oxytocin. If it possible to follow the form of the body and to vary the pressure between certain points, this contributes to tactile stimulation and can increase the positive result for the user.

It is already known to use a properly filled blanket for this purpose. Examples are also found for retail sale through catalogs or the Internet, primarily in the USA, where the use of weight blankets has been known for a long time. Popular variants show a blanket that contains plastic pellets or balls, and there are even examples of filling materials of sand, peas, gravel, metal shot, and also of popcorn! These materials all have the common feature that they are based on essentially point-like objects, and in order to keep the fillings in the desired places in blanket when it is being used, patches, preferably with a square shape, are often sewn, which are stuffed, in order to be able to lie still and exert a pressure against the body of the user, rather than running down to the lowest point of the blanket. Through such a filling, however, the blanket becomes thick, and it is difficult to regulate the temperature without removing the blanket, which can represent a significant disadvantage, especially in summertime. The blanket thus becomes relatively large and unwieldy and thereby difficult to fold up or handle, and because of the nature of the filling, cleaning and washing can be made difficult or completely impossible.

A patented weight blanket is disclosed by EP 0,729,340, where a blanket for therapeutic use has essentially square patches filled with therapy balls. The balls used have a diameter of about 50 mm, and they exert a more point-shaped pressure against the body, which is believed to increase the release of favorable bodily substances and thereby have a positive effect on the user. This, however, contributes to the blanket having limited possibilities of adjusting itself more precisely according to the user's body shape, and the blanket becomes unwieldy and difficult to handle in this case, as well. Temperature regulation becomes a problem, as in the variants of weight blankets described above.

An automobile blanket provided with chains in a grid pattern is known from US 1 364 601. It is however not intended for therapeutic treatment.

There is a need for a weight blanket that can be adjusted in a better way according to the user's body and thereby gives a better and more stimulating pressure on the user. Improvements are also needed in regard to temperature regulation and handling ability, as well as in regard to folding and washing.

### BRIEF DESCRIPTION OF THE INVENTION

One purpose of the present invention as claimed is to eliminate or at least to minimize the above-mentioned problems, which is achieved by a weight blanket for therapeutic treatment, as initially defined, wherein the linked objects create bands of weights spread over the blanket, in order for the pressure to follow the shape of the body of the user of the blanket, and in that said linked object is completely enclosed inside the blanket, and wherein said weight blanket is void of crossing linked objects of the same size.

The disclaimer included in the claims results in a restored novelty over document US 1 364 601 directed to an automobile robe and disclosing chains in a grid pattern. Belonging to a remote technical field compared to weight blankets for therapeutic use, US 1 364 601 is considered an accidental anticipation which has no relevance for assessing inventive step.

Thanks to the nature of the filling - parts linked together - a good adjustment to the shape of body can be obtained and a favorable pressure can thereby be created for the user.

According to one aspect of the invention, the at least one linked object is in the form of a chain. Through this, the linked object is held together and can be placed in a selected direction in the blanket in a simple manner. Through the chain shape, the pressure against the user can also be varied in a simple manner, since the links that constitute the chain can be linked to one another so that sometimes the wide sides and sometimes the short sides lie against one another. Further stimulation is caused by this, which can give an increased release of favorable substances in the user.

According to another aspect of the invention, said chain has a wire diameter of 1-10 mm, but preferably 2-5 mm. Thanks to this, the whole weight of the blanket can be adjusted in a simple way by choosing a chain size such that the product can be adjusted according to a specific user. Different sizes in different parts of the blanket can also be considered, which should give possibilities for further differentiated pressure over different parts of the body or different central or distal areas of the blanket. Thanks to the thin structure that a chain provides, temperature regulation can also be achieved easily, regardless of the season, since the warming properties of the blanket will arise from padding or other kinds of conventional blankets that can be combined with a weight blanket, and this application of warming material can be varied in a simple manner to adjust to the seasons and the user's desired temperature.

According to another aspect of the invention, said linked object is made of metal. Thanks to this, weight is given to the weight blanket in a simple and natural way. Metal is also a strong material that is easy to clean and handle. Thanks to the concentration of the weight in specific strands or loops, the blanket can be handled and folded easily, which would not be possible with a more uniform distribution as is required, for example, when plastic balls are used. Metal is completely non-warming as such for the user, which further simplifies temperature adjustments according to the season, the indoor climate, and the user's desires when the blanket is used over a longer period of time.

According to another aspect of the invention, said linked object is held in place in the blanket by channels. Through this the blanket can be adjusted and the linked object placed where they are considered to be able to do the greatest good.

According to one aspect of the invention, said channels run essentially parallel to the length and/or width of the blanket. Thanks to this, a weight band can be achieved that is spread over the blanket and gives the desired pressure in the form of lines along the length or the width. Through this structure, combined with the form and weight of the links, large pressure variations can be obtained, so that the user resting under the blanket does not feel limited by a static or uniform pressure but by change and stimulation of various points or lines along the body. This is believed to be especially favorable for the release of bodily substances, such as oxytocin, which contributes to a feeling of calmness and safety in the user.

According to another aspect of the invention, the ends of said linked object are attached to the blanket by fastenings in the edge of the blanket. Thanks to this, a good adhesion between the linked object and the structure of the blanket can be achieved with a few simple fastening points, and the structure of the blanket and its filling are held in place in a simple and handy manner. The fastenings can be made by riveting, which is a practical and durable method, and assure long wear and good quality even after handling that can cause wear on the blanket, for example, washing.

According to another aspect of the invention, said linked object is attached in a slit or an eye at one end of the blanket. A part of a band or another edge is made up of these slits or eyes, and fastening can thereby occur in an orderly way by a secure attachment to the blanket being formed.

According to another aspect of the invention, there is a layer of an insulating material on a first side of the surface of the blanket. Through this, the blanket as such can also have a warming function for the user without being combined with another conventional blanket being needed. The thickness and other characteristics of this insulating layer can be selected in order to adjust the warming ability of the blanket and provide the user with the type of blanket that corresponds best to his or her temperature requirements during sleep. According to yet another aspect of the invention, said linked object is placed completely on a second side of the blanket. Thereby, the user can choose to have the linked objects on the lower side of the blanket and achieve a deeper stimulation and a higher and more point- or line-shaped pressure. This gives additional possibilities for adjusting the characteristics of the blanket according to the user's needs from day to day, so that it is possible to achieve a large warming ability and versatility.

The two aspects of the invention mentioned last can be combined to give a blanket with a linked object on both sides of said insulating layer mentioned. The linked object can then be placed in various rows or patterns so that a great variation is achieved and the user can choose a pressure that is sometimes point-or line-shaped and sometimes softer and more widespread.

### BRIEF DESCRIPTION OF THE DRAWINGS

The invention will be described in more detail in the following, with reference to the attached drawings, in which:
Fig. 1 shows across-section of a weight blanket;
Fig. 2 shows an enlargement of a section of a weight blanket with a linked object attached to the blanket;
Fig. 3a shows another enlargement of the attachment of a linked object;
Fig. 3b shows a corresponding attachment according to an alternative method;
Fig. 4a shows a cross-section of a blanket according to the invention, seen in a vertical plane; and
Fig. 4b shows a cross-section of a blanket with an isolating layer according to the invention, seen in a vertical plane.

### DETAILED DESCRIPTION

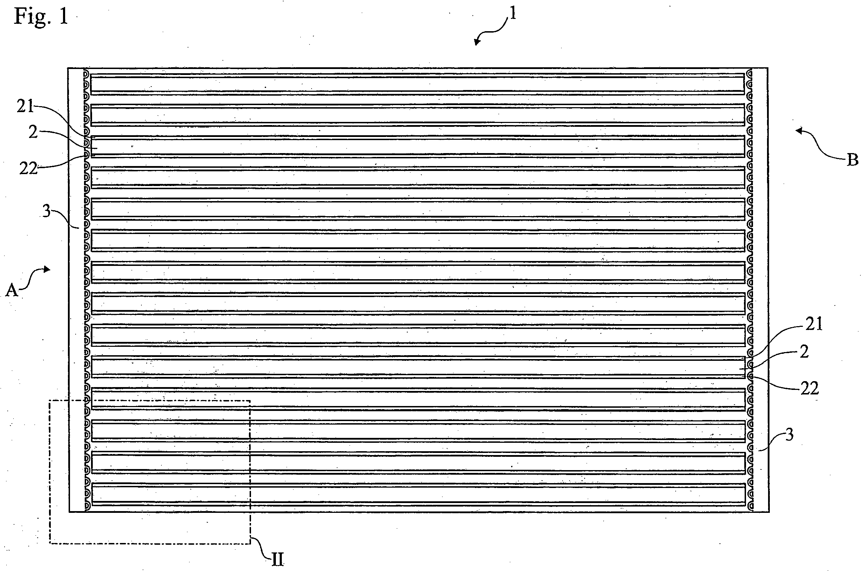

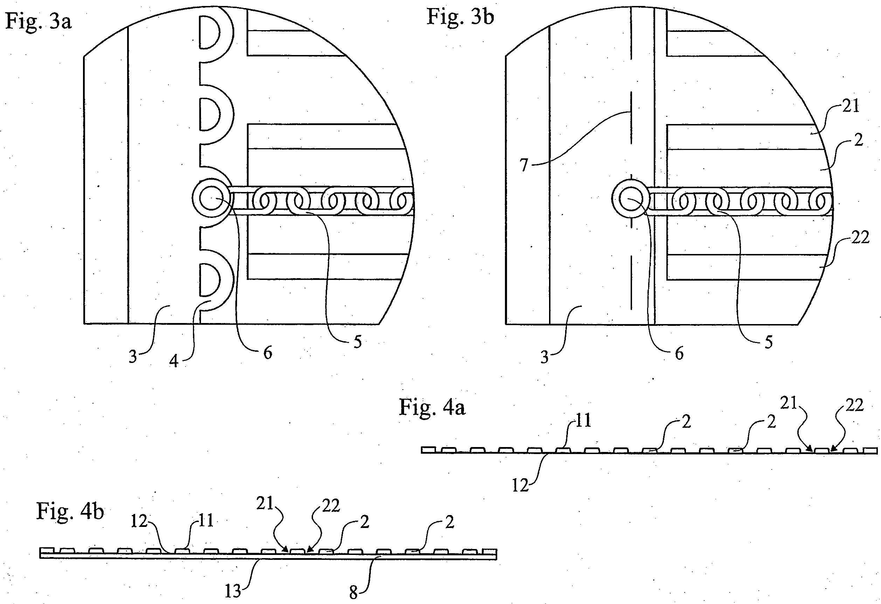

In Fig. 1, a weight blanket 1 is shown. The blanket 1 has the same dimensions as an ordinary blanket, thus a length of about 205 cm and a width of about 150 cm. The blanket 1 includes two layers of fabric 11 and 12 (shown in Figs. 4a-b), between which channels with a width of about 50 mm are made by sewing two seams 21 and 22. If necessary, these seams are doubled or tripled, in order to give firmness and smoothness to said channels 2.

The linked object 5 will be placed in the form of metal chains in the lengthwise direction of the blanket, thus from a head end A to a foot end B. In order to give simple and stable attachment, a cross band 3 transversal to the blanket is provided in both the head end A and the foot end B. For increased firmness, other bands can be placed there also, so that the ends of the blanket can become more clearly defined and the risk of damage to the blanket, as a consequence of the attached chains, can be reduced. In order to choose the type of chain that provides the best filling in every conceivable case, one needs to start by considering the user who will benefit from the blanket and from the user's weight and other characteristics and preferences, and select a wire thickness and size of the chain links that fits the user's characteristics. A wire thickness is often suitable where each link is 1-10 mm, preferably 2-5 mm thick, so that a total weight of 3-10 kg is obtained. In a blanket with the ordinary dimensions mentioned above, it might be suitable to make 30 channels in the lengthwise direction of the blanket, and in order to obtain a good pressure, it can be appropriate to place a linked object, in this case a metal chain, in every other channel 2.

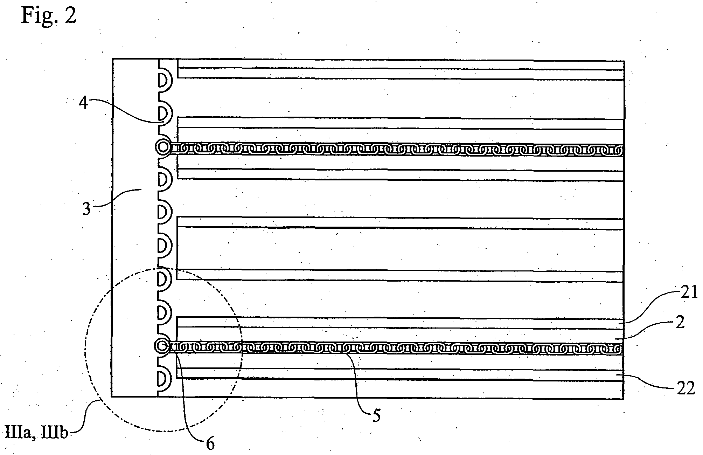

The metal chain 5 used for the blanket can be of a conventional type that can be easily acquired from a manufacturer without any special adjustment being required. In order to reduce the risk of rust or other wear, the chain can be surface-treated, for example by powder coating. The chain 5 can be placed in the desired channel 2 manually or by an automated process, and the length of the chain 5 is adjusted so that the outermost links in each direction overlap the cross-band 3 that creates firmness in the respective head and foot ends of the blanket, A, B, so that an attachment point 6 can be formed. As shown in Fig. 2, the cross-band 3 has a series of eyes of a wire that is woven into the band. In order to create a secure attachment, the outermost link of the chain 5 is placed over an eye 4 in said band 3 and attached there, preferably by riveting with a plastic rivet. An alternative
attachment method is shown in Fig. 3b, where the band 3 has lengthwise slits 7 that are woven into the band and where the outermost links of the chain 5 can be attached; here, too, preferably by riveting. This alternative attachment method has certain cost effective advantages, but both methods can give a secure attachment with good characteristics for the user.

One or more chains 5 can be used for the blanket. According to the preferred method described above, one chain per channel 2 is cut, the end links are attached to the band 3, but it can also be conceived to use a single chain that lies in loops and where the outermost links at each channel are attached to two other links that constitute the start of chains in each channel 2.

Thanks to the structure of the chain, every other eye can be placed against the user's body by its wide side, which gives a relatively low pressure and a more widespread effect, and every other eye by its short side, which gives a higher pressure and a more palpable effect.

In this preferred embodiment, the chain or chains 5 used run in a lengthwise from a head end A to a foot end B, but several other variants can also be conceived. For example, the chains 5 can run from side to side in the blanket 1, instead, or in a series of diagonals over the blanket 1. It is also conceivable to use a route pattern where the chains overlap and run in different directions. For the manufacturing, it should then be possible to use at least four cloth layers that are attached together into a final stage.

When the weight blanket 1 according to the invention is assembled so that the chains 5 are completely enclosed, it can be used as such or filled into a conventional covering, which reduces wear on the blanket itself, simplifies washing, and enables greater design variation. In order to give the desired warmth, the blanket needs to be combined with an insulating layer, such as an ordinary blanket that can be placed under or over the weight blanket. Placement under the weight blanket should distribute the pressure by which said blanket acts over a larger area of said weight blanket, while placement over the weight blanket can help the pressure from each chain to be focused on each link against the user's body.

By combination with a conventional blanket, the user can thus choose his night temperature himself through the thickness of the blanket used is and thus the experience of warmth from the blanket and by placement over or under the weight blanket, the degree and distribution of the pressure can be adjusted as needed from day to day.

In an alternative embodiment, the weight blanket is equipped directly with a layer of an insulating material, such as batting, so that a combination blanket is obtained. Here, either the chains 5 can lie completely on one side and the batting 8 on the other (shown in Fib. 4b) or it can be conceived that the batting is placed in the middle with chains on both sides or even with the chain in the middle and batting on both sides. The possibilities of variation according to each individual user's needs and preferences are thus great.

In Fig. 4a, the preferred embodiment which is also shown in Fig. 1, among others, is shown from the side, i.e., as a cross-section would appear with the blanket spread on a horizontal surface. In the drawing, the head end A is turned toward the observer, and the seams 21 and 22 that define each channel 2 can be seen. In every other one of these channels 2, chains 5 are thus placed as linked objects in accordance with the preferred embodiment as described above. Of course, a denser or thinner placement in the blanket can be conceived. In the drawing, the included cloth layers 11, 12 can also be seen, which must be at least two in number, in order to be able to enclose the linked object 5 and form channels 2.

In Fig. 4b, the alternative embodiment with a layer 8 of an insulating material such as batting is shown. Here, too, the seams 21, 22 that define each channel 2 can be seen. The at least three cloth layers 11, 12, 13 needed for the construction in order to enclose the object and the batting can also be seen.

The cloth used for the weight blanket can be a firm cotton cloth. In order to obtain a flameproof product, a cloth woven from a fireproof thread can be used, which, however, contributes to a somewhat more expensive final product.

The invention is not limited to what is described above, but it can be varied within the scope of the following claims. It can be realised, for example, that the linked object used can also be of a number of different designs, comprising both the eyes described above and other constructions. Other materials than metal can also be suitable to give the blanket the desired weight, and the linked object can be held in place in the blanket in another way, for example by the linked object being attached directly to the cloth. It can also be conceived to have the linked object lying freely in the blanket between its end points, without being limited to channels, and other attachment methods than those described can be used than just riveting.

## Claims

1. A weight blanket for therapeutic treatment, comprising means for increasing the weight of the blanket, **characterized in that** said means includes at least one linked object (5);
said at least one linked object (5) is placed in several corresponding channels (2) between a first and a second cloth layer (11, 12) in the blanket (1);
said at least one linked object (5) creating bands of weights spread over the blanket, in order for the pressure to follow the shape of the body of the user of the blanket, wherein the at least one linked object (5) is completely enclosed inside the blanket, wherein said weight blanket is void of crossing linked objects of the same size.

2. A weight blanket according to claim 1, **characterized in that** said channels are distributed over the surface of the blanket and run parallel in the lengthwise direction of the blanket.

3. A weight blanket according to claim 1 or 2, **characterized in that** the at least one linked object having a thickness in the range 1 - 10 mm, preferably 2 - 5 mm.

4. A weight blanket according to claim 1, 2 or 3, **characterized in that** said channels (2) are evenly distributed over the whole surface of the blanket.

5. A weight blanket according to any of claims 1 to 4, **characterized in that** the at least one linked object (5) is in the form of a chain.

6. A weight blanket according to any of claims 1 to 5, **characterized in that** the at least one linked object (5) is made of metal.

7. A weight blanket according to any of claims 1 to 6, **characterized in that** the ends of said the at least one linked object (5) are attached to the blanket by fastening in the edge of the blanket (3).

8. A weight blanket according to claim 7, **characterized in that** said fastening is performed by means of riveting.

9. A weight blanket according to claim 7 or 8, **characterized in that** the at least one linked object (5) is attached in a slit or eye (4) in an edge (3) of the blanket.

10. A weight blanket according to any of claims 1 to 9, **characterized in that** at least one layer of insulating material (8) is found on a first side of the surface of the blanket.

11. A weight blanket according to claim 10, **characterized in** the at least one linked object (5) being placed completely on a second side of the blanket.

12. A weight blanket according to claim 11, **characterized in that** the at least one linked object (5) is placed on both the first and the second sides of the blanket.

13. A weight blanket according to any of the preceding claims, **characterized in that** the thickness of the at least one linked object (5) is such that the weight of the blanket is in the range 3 - 10 kg.

## Patentansprüche

1. Gewichtsdecke zur therapeutischen Behandlung, welche ein Mittel zum Erhöhen des Gewichtes der Decke umfasst, **dadurch gekennzeichnet, dass** das Mittel mindestens ein verbundenes Objekt (5) beinhaltet;
wobei das mindestens eine verbundene Objekt (5) in mehreren entsprechenden Kanälen (2) zwischen einer ersten und einer zweiten Stoffschicht (11, 12) in der Decke (1) platziert ist;
wobei das mindestens eine verbundene Objekt (5) Gewichtsbänder erzeugt, die über die Decke verteilt sind, damit der Druck der Form des Körpers des Benutzers der Decke folgen kann, wobei das mindestens eine verbundene Objekt (5) vollständig im Inneren der Decke eingeschlossen ist, wobei die Gewichtsdecke frei von querlaufenden verbundenen Objekten der derselben Größe ist.

2. Gewichtsdecke nach Anspruch 1, **dadurch gekennzeichnet, dass** die Kanäle über die Fläche der Decke verteilt sind und parallel in der Längsrichtung der Decke verlaufen.

3. Gewichtsdecke nach Anspruch 1 oder 2, **dadurch gekennzeichnet, dass** das mindestens eine verbundene Objekt eine Dicke im Bereich von 1 - 10 mm, vorzugsweise 2 - 5 mm, aufweist.

4. Gewichtsdecke nach Anspruch 1, 2 oder 3, **dadurch gekennzeichnet, dass** die Kanäle (2) gleichmäßig über die gesamte Fläche der Decke verteilt sind.

5. Gewichtsdecke nach einem der Ansprüche 1 bis 4, **dadurch gekennzeichnet, dass** das mindestens eine verbundene Objekt (5) in der Form einer Kette vorliegt.

6. Gewichtsdecke nach einem der Ansprüche 1 bis 5, **dadurch gekennzeichnet, dass** das mindestens eine verbundene Objekt (5) aus Metall gemacht ist.

7. Gewichtsdecke nach einem der Ansprüche 1 bis 6, **dadurch gekennzeichnet, dass** die Enden des mindestens einen verbundenen Objekts (5) durch Befestigung im Rand der Decke (3) an der Decke angebracht sind.

8. Gewichtsdecke nach Anspruch 7, **dadurch gekennzeichnet, dass** die Befestigung mittels Vernietung erfolgt.

9. Gewichtsdecke nach einem der Ansprüche 7 oder 8, **dadurch gekennzeichnet, dass** das mindestens eine verbundene Objekt (5) in einem Schlitz oder einer Öse (4) im Rand (3) der Decke angebracht ist.

10. Gewichtsdecke nach einem der Ansprüche 1 bis 9, **dadurch gekennzeichnet, dass** sich mindestens eine Schicht aus isolierendem Material (8) auf einer ersten Seite der Fläche der Decke befindet.

11. Gewichtsdecke nach Anspruch 10, **dadurch gekennzeichnet, dass** das mindestens eine verbundene Objekt (5) komplett auf einer zweiten Seite der Decke platziert ist.

12. Gewichtsdecke nach Anspruch 11, **dadurch gekennzeichnet, dass** das mindestens eine verbundene Objekt (5) sowohl auf der ersten als auch der zweiten Seite der Decke platziert ist.

13. Gewichtsdecke nach einem der vorhergehenden Ansprüche, **dadurch gekennzeichnet, dass** die Dicke des mindestens einen verbundenen Objektes (5) derart ist, dass das Gewicht der Decke im Bereich von 3 - 10 kg liegt.

## Revendications

1. Couverture pesante pour traitement thérapeutique, comprenant des moyens pour augmenter le poids de la couverture, **caractérisée en ce que** lesdits moyens comprennent au moins un objet relié (5) ;
ledit au moins un objet relié (5) est placé dans plusieurs conduits (2) correspondants entre une première et une deuxième couches de tissu (11, 12) dans la couverture (1) ;
ledit au moins un objet relié (5) créant des bandes de poids étalées sur la couverture, de façon que la pression suive la forme du corps de l'utilisateur de la couverture, ledit au moins un objet relié (5) étant complètement enfermé à l'intérieur de la couverture, ladite couverture pesante étant dépourvue d'objets reliés croisés de la même taille.

2. Couverture pesante selon la revendication 1, **caractérisée en ce que** lesdits conduits sont répartis sur la surface de la couverture et s'étendent parallèlement dans le sens de la longueur de la couverture.

3. Couverture pesante selon la revendication 1 ou la revendication 2, **caractérisée en ce que** ledit au moins un objet relié présente une épaisseur comprise entre 1 et 10 mm, de préférence entre 2 et 5 mm.

4. Couverture pesante selon la revendication 1, 2 ou 3, **caractérisée en ce que** lesdits conduits (2) sont répartis uniformément sur toute la surface de la couverture.

5. Couverture pesante selon l'une quelconque des revendications 1 à 4, **caractérisée en ce que** ledit au moins un objet relié (5) se présente sous la forme d'une chaîne.

6. Couverture pesante selon l'une quelconque des revendications 1 à 5, **caractérisée en ce que** ledit au moins un objet relié (5) est réalisé en métal.

7. Couverture pesante selon l'une quelconque des revendications 1 à 6, **caractérisée en ce que** les extrémités dudit au moins un objet relié (5) sont fixées à la couverture par fixation au niveau du bord de la couverture (3).

8. Couverture pesante selon la revendication 7, **caractérisée en ce que** ladite fixation est réalisée par rivetage.

9. Couverture pesante selon la revendication 7 ou la revendication 8, **caractérisée en ce que** ledit au moins un objet relié (5) est fixé dans une fente ou un oeillet (4) situé au niveau d'un bord (3) de la couverture.

10. Couverture pesante selon l'une quelconque des revendications 1 à 9, **caractérisée en ce qu'**au moins une couche de matériau isolant (8) se trouve sur un premier côté de la surface de la couverture.

11. Couverture pesante selon la revendication 10, **caractérisée en ce que** ledit au moins un objet relié (5) est placé complètement sur un second côté de la couverture.

12. Couverture pesante selon la revendication 11, **caractérisée en ce que** ledit au moins un objet relié (5) est placé à la fois sur les premier et deuxième côtés de la couverture.

13. Couverture pesante selon l'une quelconque des revendications précédentes, **caractérisée en ce que** l'épaisseur dudit au moins un objet relié (5) est telle que le poids de la couverture est situé dans l'intervalle allant de 3 à 10 kg.
